# EUROPEAN PATENT SPECIFICATION

(11) **EP 3 904 329 B1**
(45) Date of publication and mention of the grant of the patent: **04.09.2024**
(21) Application number: 21170571.0
(22) Date of filing: 26.04.2021
(51) Int. Cl.: C07C 209/74, C07C 209/48, C07C 201/12, C07C 211/47, C07C 211/27, C07C 211/45, C07C 205/22, C07C 37/50, C07C 51/377, C07C 39/28, C07C 63/04

(54) **PROCESS FOR HYDRODEFLUORINATION OF AROMATIC TRIFLUOROMETHYL DERIVATIVES**
VERFAHREN ZUR HYDRODEFLUORIERUNG VON AROMATISCHEN TRIFLUORMETHYLDERIVATEN
PROCÉDÉ DE HYDROFLUORATION DE DÉRIVÉS AROMATIQUES DE TRIFLUOROMÉTHYL

(30) Priority: 01.05.2020 CZ 20200246
(43) Date of publication of application: 03.11.2021
(73) Proprietor: Univerzita Pardubice, 530 09 Pardubice, Polabiny (CZ)
(72) Inventor: Weidlich, Tomas, 53341 Lazne Bohdanec (CZ)
(74) Representative: Hartvichova, Katerina

(56) References cited:
- TAN SONG-LIANG ET AL: "Raney Ni-Al alloy-mediated reduction of alkylated phenols in water", JOURNAL OF CHEMICAL RESEARCH, 1 January 2009 (2009-01-01), pages 5 - 7, XP055838222, Retrieved from the Internet <URL:https://journals.sagepub.com/doi/pdf/10.3184/030823409X393637> [retrieved on 20210906]
- TOMÃ Å WEIDLICH ET AL: "Facile dehalogenation of halogenated anilines and their derivatives using Al-Ni alloy in alkaline aqueous solution", CENTRAL EUROPEAN JOURNAL OF CHEMISTRY, CENTRAL EUROPEAN SCIENCE JOURNALS, PL; US; NL; DE, vol. 9, no. 4, 12 April 2011 (2011-04-12), pages 590 - 597, XP019912113, ISSN: 1644-3624, DOI: 10.2478/S11532-011-0033-7

## Description

### Field of Art

The present invention relates to the chemical degradation of aromatic compounds comprising a CF₃ substituent, by means of reductive defluorination of aromatic trifluoromethyl derivatives in alkaline aqueous medium under Ni catalysis.

### Background Art

Trifluoromethylated aromatic compounds belong amongst industrially important compounds used for the production of organic pigments, pesticides and drugs. However, trifluoromethylated aromates are biologically active (toxic) compounds which, when they contaminate water, increase the COD parameter and the content of organically bound fluorine, while being very poorly biodegradable or not biodegradable at all.

Chemical oxidation processes are often used to decompose difficult-to-biodegrade aromatic compounds in wastewater. However, efficient removal of aromatic trifluoromethyl derivatives by chemical oxidation methods requires the application of large excesses of these oxidizing agents, as the C-H and C-C bonds tend to be much more prone to oxidation than carbon-fluorine (generally carbon-halogen) bonds. This results in unbearably high costs for a sufficiently efficient chemical oxidation process, with a large excess of oxidizing agents (usually up to 100 times the stoichiometry) required to cleave the C-F bonds of the contaminants, leading to complete mineralization of organic contaminants to inorganic oxidation products (carbon dioxide, water, hydrogen halides or salts thereof, etc.), as described, for example, in: Bruton T.A., Sedlak D.L. Treatment of Aqueous Film-Forming Foam by Heat-Activated Persulfate Under Conditions Representative of In Situ Chemical Oxidation. Environmental Science & Technology, 51(23) (2017) 13878-13885.

Reductive techniques, on the other hand, lead to conversion of aromatic fluorinated derivatives into defluorinated organic products, which are usually less toxic (biologically less active) and significantly more biodegradable, allowing a much smaller excess of reducing agents to be used, and subsequent use of the cheapest biological degradation processes by common techniques used in wastewater treatment plants.

Therefore, the use of reductive processes is a very efficient and economically interesting technique for the degradation of aromatic trifluoromethyl derivatives. Its main advantage is its high selectivity (under appropriately selected reaction conditions only carbon-halogen bond cleavage occurs), which allows the use of only a small excess of effective reducing agent for the desired hydrodefluorination.

Currently, processes based on the application of base metals in the form of nanoparticles are also being investigated for hydrodefluorination of polyfluorinated derivatives. Nanoparticles of base metals (e.g. iron) hydrodefluorinate the fluorinated derivatives only very slowly and with low conversion rates, therefore it is necessary to use an enormous excess for efficient hydrodefluorination, as described e.g. in Arvaniti, O.S., et al.: Reductive degradation of perfluorinated compounds in water using Mg-aminoclay coated nanoscale zero valent iron. Chemical Engineering Journal, 262, (2015) 133-139.

For reductive hydrodefluorination, processes in anhydrous aprotic solvents are described using hydrides of elements of groups 13 and 14 (especially organometallic compounds of silicon, tin, aluminum). To carry out this reaction it is often necessary to use expensive catalysts based on platinum metals (e.g. Sabater, Sara et al: Hydrodefluorination of carbon-fluorine bonds by the synergistic action of a ruthenium-palladium catalyst. Nature Communications, 4, 3553, 2013), alternatively, nickel coordination compounds are also reported as catalytically active (e.g. LaPierre, Etienne A. et al.: Redox-state dependent activation of silanes and ammonia with reverse polarity (PCcarbeneP)Ni complexes: electrophilic vs. nucleophilic carbenes. Dalton Transactions, 47(46) (2018) 16789-16797), or Lewis acids of unusual structure (Chitnis, S.S. et al: Catalytic Hydrodefluorination of C-F Bonds by an Air-Stable PIII Lewis Acid. Chemistry - A European Journal, 24(25) (2018) 6543-6546.; Hayatifar, Ardalan et al: Transition metal-free hydrodefluorination of acid fluorides and organofluorines by Ph3GeH promoted by catalytic [Ph3C][B(C6F5)4]. Chemical Communications (Cambridge, United Kingdom), 55(73) (2019) 10852-10855).

Journal of Chemical Research, 2009, pages 5-7 describes the preparation of p-cresol from trifluoromethylphenol using Raney Ni-Al alloy in an alkaline solution, wherein mostly hydrolysis of CF3-group occurs with only minor occurence of hydrodefluorination.

All of the above hydrodefluorination processes are very far from the possibility of practical use for decontamination processes.

Thus, there remains a need to find a process for hydrodefluorination of aromatic trifluoromethyl derivatives that is economically effective, provides biodegradable products, and is efficient.

### Disclosure of the Invention

Object of the present invention is a method of hydrodefluorination (i.e. reductive cleavage of C-F bond) of aromatic trifluoromethyl compounds of formula I 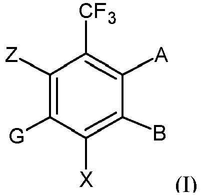
wherein A, B, X, G and Z are independently selected from the group comprising H, I, Br, F, Cl, CF₃, COOR, NO₂, N=N-R, SO₃R, CH₂R, OR, NR₂, CH₂NR₂, R-C=O, CN;
and wherein R is H, C₁-C₈ alkyl, C₆-C₁₄ aryl, or C₂-C₁₀ heterocyclyl containing one to four heteroatoms independently selected from O, S, N;
in alkaline aqueous environment, comprising the steps of:
   a) adding to an aqueous or colloid solution of the compound of formula I, at temperature within the range of 0 to 100 _{°}C a mixture comprising:
      - component A, which is an alloy containing aluminium (Al) acting as reducing agent and nickel (Ni) acting as hydrodefluoration catalyst, wherein the molar ratios of CF₃ to Al and Ni are CF₃ : Al within the range of 1 mol of CF₃ to 10-75 mol Al, and CF₃ : Ni within the range of 1 mol CF₃ to 4.6-35 mol Ni;
      - component B, which is at least one alkali metal hydroxide or carbonate in molar ratio to Al (Al which forms part of the component A) 2:1 to 6:1, wherein the amount used corresponds to pH of the reaction mixture higher than 11;
   b) stirring the resulting reaction mixture (in the form of suspension) at a temperature within the range of 10 to 100 _{°}C, preferably at a temperature within the range of 20 to 30 _{°}C, for at least 60 minutes and at most 24 hours (preferably 4 to 16 hours);
   c) after completion of stirring, separating the nickel sludge, e.g. by sedimentation and subsequent filtration or centrifugation;
   d) adjusting pH of the separated aqueous phase of the reaction mixture to a value within the range of 6 to 9, preferably 6 to 7, which leads to precipitation of insoluble aluminium salts which adsorb microparticles of nickel and organic compounds from the mixture, separating the precipitate, e.g. by sedimentation and/or filtration or centrifugation.

The resulting aqueous phase contains less than 5 % of the initial amount of the compound of formula I and organic compounds comprising C-F bonds.

The component A is preferably Raney Al-Ni alloy with weight ratio of Al:Ni = 1:1, i.e. 50 wt.% Al and 50 wt.% Ni, or nickel bronze, alloys containing the metals CuAlNiFe and optionally Mn, preferably with molar ratio Al:Ni = 2:1 or higher, such as CuAl₁₀Ni₅Fe₄.

The component A is preferably added in smaller portions, for example in two or three or four or five portions.

In some embodiments, in addition to components A and B, an auxiliary reducing agent (component C) is added to the mixture in step (a), in order to reduce other reducible functional groups than CF₃, which may be contained in the compound of formula I (such as C-halogen bonds wherein halogen is other than F, - NO₂, -N=N-, carbonyl group, alkene (-C=C-)). Use of the auxiliary reducing agent allows to achieve a complete reduction of the compound of formula I at lower ratios of component A to the compound of formula I, because the reducing agent A is not consumed by reducing other than C-F bonds.

When the auxiliary reducing agent (component C) is used, any water-soluble base (component B) selected from alkali metal hydroxides and/or carbonates can be used. When no auxiliary reducing agent is used, use of alkali metal hydroxide as component B results in a higher effectiveness.

Component C, the auxiliary reducing agent, is preferably selected from the group comprising tetrahydroborates (e.g. NaBH₄, KBH₄), secondary C3-C6 alcohols (e.g. isopropyl alcohol, 2-hydroxy-1,3-dioxolane), elemental aluminium, technical grade iron (including nanoFe), ferrous(II) sulfate, ferrous(II) chloride, magnesium, Devarda's Al-Cu-Zn alloy (with content of 45-50 wt.% Al, 3-5 wt.% Zn and 45-50 wt.%Cu), Arnd's alloy Cu-Mg (with content of 40-50 wt.% Cu and 50-60 wt.% Mg), hydrazine and salts thereof with formic, sulfuric or hydrochloric acids, phosphinic acid and salts thereof with alkali metals, and/or reducing saccharides (e.g. glucose).

In some embodiments, step (a) is preceded by a pre-treatment step comprising reduction of other reducible functional groups than CF₃, which may be contained in the compound of formula I (such as C-halogen bonds wherein halogen is other than F, -NO₂, -N=N-, carbonyl group, alkene (-C=C-)) by applying the component C together with an undissolved (water-insoluble) part of once used (i.e., recycled) component A. The recycled component A typically contains predominantly Ni.

The pre-treatment step may be carried out instead of adding the component C in the step (a), or in addition to adding the component C to the mixture in the step (a).

Component B (base) is preferably selected from the group comprising sodium hydroxide, potassium hydroxide, sodium carbonate, potassium carbonate, and mixtures thereof.

Preferably, the pH value of the separated aqueous phase of the reaction mixture is adjusted to pH in the range of 6 to 9, using a acidic-reacting agent selected from the group comprising saturation by gas mixtures comprising CO₂ (e.g. flue gas separated from the solids), aqueous solution of sulfuric acid, aqueous solution of hydrochloric acid, aqueous solution of phosphoric acid, or aqueous solution of nitric acid.

The invention thus provides a procedure of AOX dehalogenation using a reducing agent, under catalysis by nickel prepared in situ from the component A, e.g., from Raney Al-Ni alloy.

(subst.)Ar-CF₃ + n Al-Ni + 3n OH⁻ + n H₂O → (subst.)Ar-CH₃ + n Al(OH)₄⁻ + 3 F⁻

Dissolved aluminium is removed from the aqueous phase together with nickel microparticles by means of neutralization (or slight acidification) to pH = 6 to 9, preferably 6 to 7, according to the following equation:

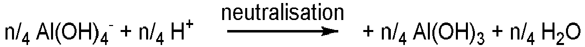

Within the framework of the present invention, it was found that this process leads to a complete reductive defluoration of compounds of formula I, and simultaneously leads to a complete removal of the unreacted agents. The process may easily be scaled up.

### Examples of carrying out the Invention

Filtration was performed using KA 4 filter paper, manufactured by Papirna Pernštejn, s.r.o., or using filter cloth PP010308 produced by ECE Group.

Comparative examples show the use of either Raney nickel with an alternative reducing agent or an attempt to replace the Al-Ni alloy by an alternative hydrodefluorinating agent.

Data shown in % are given in % by weight, unless otherwise stated.

### Example 1:

To 100 ml of aqueous solution containing 5 mmol/l of 2-chloro-4-(trifluoromethyl)aniline and 1 mol/l of isopropyl alcohol was added 0.54 g of Raney Al-Ni alloy and 50 ml of aqueous 1 mol/l NaOH solution with vigorous stirring. After stirring for 30 minutes, a second portion of 0.27 g of Al-Ni alloy was added, and after additional 30 minutes of stirring, a third portion 0.27 g of Al-Ni alloy was added. After stirring for further 60 minutes, further 50 ml of 1 mol/l NaOH was added to the suspension, and the suspension was stirred for 8 hours.

A portion of the filtrates (100 ml) was extracted with dichloromethane (3x 25 ml CH₂Cl₂). Subsequently, the dichloromethane extract was concentrated and evaporated to dryness. According to ¹H and ¹⁹F NMR analysis of the residue, quantitatively dissolved in hexadeuteriosulfoxide, this residue of the dichloromethane extract contains only 4-methylaniline (p-toluidine, i.e. pure product of hydrodefluorination).

The remaining filtrates were neutralized with 85% phosphoric acid to pH = 6.86, then subjected to ICP-OES analysis, which showed a residual Ni content below 0.1 mg Ni/l and an Al content below 0.5 mg Al/l.

### Example 2 - comparative example:

To 100 ml of aqueous solution containing 5 mmol/l of 2-chloro-4-(trifluoromethyl)aniline and 1 mol/l of isopropyl alcohol, 0.54 g of Devarda's Al-Cu(Zn) alloy and 50 ml of 1 mol/l aqueous NaOH solution were added with vigorous stirring. After stirring for 30 minutes, a second portion of 0.27 g of Devarda's Al-Cu(Zn) alloy was added, and after additional 30 minutes of stirring, a third portion of 0.27 g of Devarda's Al-Cu(Zn) alloy was added. After stirring for further 60 minutes, further 50 ml of 1 mol/l NaOH was added to the suspension, and the suspension was stirred for 8 hours.

A portion of the filtrates (100 ml) was extracted with dichloromethane (3x 25 ml CH₂Cl₂). Subsequently, the dichloromethane extract was concentrated and evaporated to dryness. According to ¹H and ¹⁹F NMR analysis of the residue quantitatively dissolved in hexadeuteriosulfoxide, this residue of the dichloromethane extract contains only unreacted 2-chloro-4-(trifluoromethyl)aniline.

The remaining filtrates were neutralized with 85% phosphoric acid to pH = 6.86, then subjected to ICP-OES analysis, which showed a residual Cu content below 0.1 mg Cull, Al content below 0.5 mg Al/l and Zn content below 1 mg Zn/l.

### Example 3:

To 100 ml of aqueous solution containing 5 mmol/l of 2,6-dichloro-4-(trifluoromethyl)aniline and 1 mol/l of isopropyl alcohol, 0.54 g of Raney Al-Ni alloy and 50 ml of 1 mol/l aqueous NaOH solution were added with vigorous stirring. After stirring for 30 minutes, a second portion of 0.27 g of Al-Ni alloy was added, followed by a third portion of 0.27 g of Al-Ni alloy after an additional 30 minutes of stirring, this was repeated a total of 3 times. After stirring for further 60 minutes, further 80 ml of 1 mol/l NaOH were added to the suspension and the suspension was stirred for 8 hours.

A portion of the filtrates (100 ml) was further extracted with dichloromethane (3x 50 ml CH₂Cl₂). Subsequently, the dichloromethane extract was concentrated and evaporated to dryness. According to ¹H and ¹⁹F NMR analysis of the residue quantitatively dissolved in hexadeuteriosulfoxide, this residue of the dichloromethane extract contains only 4-methylaniline (p-toluidine, i.e. pure product of hydrodefluorination).

The remaining filtrates were neutralized with 16% sulfuric acid to pH = 6.9, then subjected to ICP-OES analysis, which showed a residual Ni content below 0.1 mg Ni/l and an Al content below 0.5 mg Al/l.

### Example 4:

To 100 ml of aqueous solution containing 10 mmol/l of 3-(trifluoromethyl)aniline, 0.81 g of Raney Al-Ni alloy and after stirring 30 ml of 1 mol/l aqueous NaOH solution were added during 30 seconds with vigorous stirring (i.e., a total of 30 mmol NaOH). After stirring for 4 hours, a second portion of 40 ml of 1 mol/l aqueous NaOH solution was added. After 12 hours of stirring, 50 ml of dichloromethane was added, the suspension was stirred and separated by sedimentation. After the separation, the dichloromethane phase was taken for analysis of aromatic compounds content. The sedimented solid phase was extracted 3 more times with 25 ml of CH₂Cl₂ each time, the liquid phase was always added to the aqueous phase forming the upper layer in a separatory funnel, the liquid phases were shaken vigorously for 5 minutes and the lower dichloromethane phase was separated and the dichloromethane extracts were collected. Subsequently, the combined dichloromethane extracts were concentrated and evaporated to dryness. According to ¹H and ¹⁹F NMR analysis of the residue quantitatively dissolved in deuteriochloroform, this residue of dichloromethane extract contains 3-methylaniline.

The aqueous phase was neutralized with 16% sulfuric acid to pH = 6.9, then the resulting precipitate was filtered off and the filtrates were subjected to ICP-OES analysis, which showed a residual Ni content below 0.1 mg Ni/l and an Al content below 0.5 mg Al/l.

### Example 5:

To 100 ml of an aqueous solution containing 10 mmol/l of 2-chloro-4-(trifluoromethyl)phenol and 100 mol/l ofNaOH were added, with vigorous stirring, 0.55 g of Raney Al-Ni alloy and 5.4 g of a commercial aqueous solution of NaBH₄ (content 12 wt.%) in 14 MNaOH. The resulting suspension was further stirred for 2 hours and then warmed to 80 °C over 90 minutes. After cooling, 50 ml of dichloromethane were added to the suspension, followed by acidification of the reaction mixture with 16% aqueous sulfuric acid solution, and the resulting suspension was stirred and separated by sedimentation. After separating the liquid phase, the dichloromethane layer was separated for analysis of aromatics content. The sedimented solid phase was extracted 3 times with 25 ml of CH₂Cl₂, the liquid phases were added to the aqueous phase forming the upper layer in a separatory funnel. The liquid phases were shaken vigorously for 5 minutes each time and the separated dichloromethane phases were collected. Subsequently, the combined dichloromethane extracts were concentrated and evaporated to dryness. According to ¹H and ¹⁹F NMR analysis of the residue quantitatively dissolved in hexadeuteriosulfoxide, this dichloromethane extract residue contains only organic hydrodefluorination products which do not give any signal in the ¹⁹F NMR spectrum.

### Example 6 - comparative example:

To 100 mL of an aqueous solution containing 10 mmol/l 2-chloro-4-(trifluoromethyl)phenol and 100 M NaOH was added, under vigorous stirring, 0.67 g of a 50% aqueous suspension of Raney nickel (supplied by Riedel-de Haen), 0.27 g of aluminum powder and 0.67 g of Devarda's Al-Cu-Zn alloy. 9 ml (12.4 g) of a commercial aqueous solution containing 12% by weight of NaBH₄ in 14 mol/l NaOH was added to the resulting suspension, and the suspension was further stirred for 8 hours.

50 ml of dichloromethane were added to the suspension, followed by acidification of the reaction mixture with 16% aqueous sulfuric acid solution, the resulting suspension was stirred and separated by sedimentation. After removing the liquid phase, the dichloromethane layer was separated for analysis of aromatics content. The sedimented solid phase was extracted 3 times with 25 ml of CH₂Cl₂, the liquid phase was added to the aqueous phase forming the upper layer in a separatory funnel, the liquid phases were shaken vigorously for 5 minutes and the separated dichloromethane phases were collected.

Subsequently, the combined dichloromethane extracts were concentrated and evaporated to dryness. According to ¹H and ¹⁹F NMR analysis of the residue quantitatively dissolved in hexadeuteriosulfoxide, this residue of dichloromethane extract contains only unreacted 2-chloro-4-(trifluoromethyl)phenol and 4-(trifluoromethyl)phenol, in the ¹⁹F NMR spectrum there are also 2 signals having chemical shifts -61.75 ppm and -68.36 ppm.

### Example 7:

To 100 ml of an aqueous solution containing 10 mmol/l of 4-nitro-3-(trifluoromethyl)phenol and 100 mol/l of NaOH was added, with vigorous stirring, 70 ml of 1M aqueous NaOH solution and then dropwise 30 ml of an aqueous solution of FeSO₄ at a concentration of 0.5 mol/l. The resulting green suspension was stirred in the reaction vessel for 2 hours, then 0.54 g of Raney Al-Ni alloy was added and the resulting suspension was further stirred for 1 hour and then heated to 80 °C over 90 minutes. After the reaction mixture was cooled, 50 ml of dichloromethane was added to the suspension, followed by acidification of the reaction mixture with 16% aqueous sulfuric acid solution, and the resulting suspension was stirred and separated by sedimentation. After removing the liquid phase, the dichloromethane layer was separated for analysis of aromatics content. The sedimented solid phase was extracted 3 times with 25 ml of CH₂Cl₂, the liquid phase was added to the aqueous phase forming the upper layer in a separatory funnel, the liquid phases were shaken vigorously for 5 minutes and the separated dichloromethane phases were collected. Subsequently, the combined dichloromethane extracts were concentrated and evaporated to dryness. According to ¹H and ¹⁹F NMR analysis of the residue quantitatively dissolved in hexadeuteriosulfoxide, this dichloromethane extract residue contains only organic products which do not give any signal in the ¹⁹F NMR spectrum.

### Example 8:

To 100 ml of an aqueous solution containing 10 mmol/l of 4-nitro-3-(trifluoromethyl)phenol and 100 mol/l of NaOH was added, with vigorous stirring, 70 ml of 1M aqueous NaOH solution and then dropwise 30 ml of an aqueous solution of FeSO₄ at a concentration of 0.5 mol/l, and the resulting green suspension was stirred in the reaction vessel for 2 hours. Then 0.54 g of Raney Al-Ni alloy was added and the resulting suspension was further stirred for 1 hour and then tempered to 80 °C over 90 minutes. After the reaction mixture was cooled, 50 ml of dichloromethane was added to the suspension, followed by acidification of the reaction mixture with 16% aqueous sulfuric acid solution, and the resulting suspension was stirred and separated by sedimentation. After removing the liquid phase, the dichloromethane layer was separated for analysis of aromatics content. The sedimented solid phase was extracted 3 times with 25 ml of CH₂Cl₂, the liquid phase was added to the aqueous phase forming the upper layer in a separatory funnel, the liquid phases were shaken vigorously for 5 minutes and the separated dichloromethane phases were collected. Subsequently, the combined dichloromethane extracts were concentrated and evaporated to dryness. According to ¹H and ¹⁹F NMR analysis of the residue quantitatively dissolved in hexadeuteriosulfoxide, this dichloromethane extract residue contains only organic products which do not yield any signal in the ¹⁹F NMR spectrum.

### Example 9:

To 100 ml of an aqueous solution containing 10 mmol/l of 4-nitro-3-(trifluoromethyl)phenol and 100 mol/l of NaOH was added, with vigorous stirring, 80 ml of 1M aqueous NaOH solution and 2 g of glucose monohydrate, the solution was heated to 90 °C for 2 hours. The solution was then cooled to 40 °C, and 0.54 g of Raney Al-Ni alloy was added thereto, and the resulting suspension was further stirred for 1 hour and then heated to 80 °C over 90 minutes. After cooling the reaction mixture, 50 ml of dichloromethane was added to the suspension, followed by acidification of the reaction mixture with 16% aqueous sulfuric acid solution, and the resulting suspension was stirred and separated by sedimentation. After removing the liquid phase, the dichloromethane layer was separated for analysis of aromatics content. The sedimented solid phase was extracted 3 times with 25 ml of CH₂Cl₂, the liquid phase was added to the aqueous phase forming the upper layer in a separatory funnel, the liquid phases were shaken vigorously for 5 minutes and the separated dichloromethane phases were collected. Subsequently, the combined dichloromethane extracts were concentrated and evaporated to dryness. According to ¹H and ¹⁹F NMR analysis of the residue quantitatively dissolved in hexadeuteriosulfoxide, this dichloromethane extract residue contains only organic products which do not yield any signal in the ¹⁹F NMR spectrum.

### Example 10:

To 100 ml of an aqueous solution containing 5 mmol/l of 2-fluoro-6-(trifluoromethyl)benzylamine and 1 mol/l of isopropyl alcohol, 0.81 g of Raney Al-Ni alloy and 50 ml of aqueous 1 mol/l NaOH solution were added with vigorous stirring. After stirring for 30 minutes, a second portion of 0.14 g of Al-Ni alloy was added, and after an additional 30 minutes of stirring, a third portion 0.14 g of Al-Ni alloy was added. After stirring for further 60 minutes, further 50 ml of 1 mol/l NaOH were added to the suspension and the suspension was further stirred for 8 hours.

Subsequently, after cooling, the filtrates obtained were extracted with dichloromethane (3x 25 ml CH₂Cl₂). Subsequently, the dichloromethane extract was concentrated and evaporated to dryness. According to ¹H and ¹⁹F NMR analysis of the residue quantitatively dissolved in hexadeuteriosulfoxide, this residue of the dichloromethane extract contains 2-methyl-benzylamine, i.e. the hydrodefluorination product.

The remaining aqueous phase after extraction with dichloromethane was neutralized with 16% sulfuric acid (obtained by mixing 1 volume of concentrated sulfuric acid with 10 parts of water) to pH = 6.7, the filtrates obtained by separating the formed precipitate were subsequently subjected to ICP-OES analysis showing the residual content of Ni below 0.1 mg Ni/l and the Al content below 0.5 mg Al/l.

### Example 11:

To 100 ml of an aqueous solution containing a mixture of 5 mmol/l 4-CF₃-benzoic acid and 5 mmol/l 2-CF₃-benzoic acid in 20 mmol/l KOH was added 0.55 g of Raney Al-Ni alloy and 30 ml of aqueous 1 mol/l KOH solution. After stirring for 30 minutes, a second portion of 0.14 g of Al-Ni alloy was added to the reaction mixture, followed by an additional 30 ml of a 1 mol/l aqueous NaOH solution, and the suspension was further stirred for 4 hours.

Subsequently, after cooling, the filtrates obtained were acidified with 16% sulfuric acid to pH = 2-3, extracted with dichloromethane (3x 50 ml CH₂Cl₂). Subsequently, the dichloromethane extract was concentrated and evaporated to dryness. According to ¹H and ¹⁹F NMR analysis of the residue quantitatively dissolved in hexadeuteriosulfoxide, this residue of the dichloromethane extract contains 2-methylbenzoic acid and 4-methylbenzoic acid, i.e. hydrodefluorination products.

### Example 12:

To 100 ml of a 1:1 water: isopropyl alcohol solution containing 10 mmol/l of 2-chloro-5-(trifluoromethyl)aniline, 4 g of a commercial aqueous solution of NaBH₄ containing 12% by weight of NaBH₄ in 14 mol/l NaOH were added with vigorous stirring. The mixture was stirred and then 0.81 g of Raney Al-Ni alloy was added. After stirring for further 12 hours, 50 ml of dichloromethane were added to the reaction mixture, the suspension was stirred and separated by sedimentation. After removing the liquid phase, the dichloromethane layer was separated for analysis of aromatics content. The sedimented solid phase was extracted 3 times with 25 ml of CH₂Cl₂, the liquid phase was added to the aqueous phase forming the upper layer in a separatory funnel, the liquid phases were shaken vigorously for 5 minutes and the separated dichloromethane phases were collected. Subsequently, the dichloromethane extract was concentrated and evaporated to dryness. According to ¹H and ¹⁹F NMR analysis of the residue quantitatively dissolved in deuteriochloroform, this residue of the dichloromethane extract contains 3-methylaniline.

The aqueous phase was neutralized with 16% sulfuric acid to pH = 6.9, then the resulting precipitate was filtered off and the filtrates were subjected to ICP-OES analysis, which showed the residual Ni content below 0.1 mg Ni/l and the Al content below 0.5 mg Al/l.

### Example 13:

To 100 ml of an aqueous solution containing 10 mmol/l of 3-(trifluoromethyl)aniline, 0.81 g of Raney Al-Ni alloy was added with vigorous stirring. After thorough stirring, 6.9 g of K₂CO₃ was added in one portion and the mixture was heated to 60 °C, then the heating was stopped. After stirring for further 6 hours, 50 ml of dichloromethane were added to the reaction mixture, the suspension was stirred and separated by sedimentation. After removing the liquid phase, the dichloromethane layer was separated for analysis of aromatics content. The sedimented solid phase was extracted 3 times with 25 ml of CH₂Cl₂, the liquid phase was added to the aqueous phase forming the upper layer in a separatory funnel, the liquid phases were shaken vigorously for 5 minutes and the separated dichloromethane phases were collected. Subsequently, the dichloromethane extract was concentrated and evaporated to dryness. According to ¹H and ¹⁹F NMR analysis of the residue quantitatively dissolved in deuteriochloroform, this residue of the dichloromethane extract contains 3-methylaniline.

The aqueous phase was neutralized with 16% sulfuric acid to pH = 6.9, then the resulting precipitate was filtered off and the filtrates were subjected to ICP-OES analysis, which showed the residual Ni content below 0.1 mg Ni/l and the Al content below 0.5 mg Al/l.

### Example 14:

To the insoluble part of reaction mixture from Example 11, 100 ml of an aqueous solution containing 10 mmol/l of 4-hydroxy-3-(trifluoromethyl)benzonitrile and 20 mol/l of NaOH was added with vigorous stirring. Subsequently, 10 mmol of hydrazine hydrate and 25 mmol of formic acid was added under vigorous stirring. The reaction mixture was stirred in the reaction vessel for 2 hours over 95 _{°}C under reflux, then cooled below 50 °C and 50 ml of aqueous 1 mol/l NaOH and 0.54 g of Raney Al-Ni alloy were added under vigorous stirring. The resulting suspension was further stirred for 2 hours and then heated to 80 °C over 90 minutes. After the reaction mixture was cooled, 50 ml portion of reaction mixture was collected. Subsequently, 50 ml of dichloromethane was added to the collected sample, followed by acidification of the reaction mixture with 16% aqueous sulfuric acid solution, and the resulting suspension was stirred and separated by sedimentation. After removing the liquid phase, the dichloromethane layer was separated for analysis of aromatics content. The sedimented solid phase was extracted 3 times with 25 ml of CH₂Cl₂, the liquid phase was added to the aqueous phase forming the upper layer in a separatory funnel, the liquid phases were shaken vigorously for 5 minutes and the separated dichloromethane phases were collected. Subsequently, the combined dichloromethane extracts were concentrated and evaporated to dryness. According to ¹H and ¹⁹F NMR analysis of the residue quantitatively dissolved in hexadeuteriosulfoxide, this dichloromethane extract residue contains only organic product 4-hydroxy-3-methyl-benzylamine which do not give any signal in the ¹⁹F NMR spectrum.

### Example 15:

To the insoluble part of reaction mixture from Example 10, 100 ml of solution containing 10 mmol/l of 3'-(trifluoromethyl)acetophenone dissolved in 50 vol.% aqueous methanol and subsequently 10 mmol of powdered NaBH₄ was added under vigorous stirring and stirred under heating at 60-65°C for 2 hours. Subsequently, after cooling to 40 _{°}C, 0.55 g of Raney Al-Ni alloy and 30 ml of aqueous 1 mol/l KOH solution were added. After stirring for 120 minutes, a second portion of 0.14 g of Raney Al-Ni alloy was added to the reaction mixture, followed by an additional 30 ml of a 1 mol/l aqueous NaOH solution, and the suspension was further stirred for 4 hours.

Subsequently, after sedimentation, deccantation and filtration of liquid phase, the obtained filtrate were acidified with 16 wt.% sulfuric acid to pH = 2-3, extracted with dichloromethane (3x 50 ml CH₂Cl₂). Subsequently, the dichloromethane extract was concentrated and evaporated to dryness. According to ¹H and ¹⁹F NMR analysis of the residue quantitatively dissolved in hexadeuteriosulfoxide, this residue of the dichloromethane extract contains hydrodefluorinated products.

## Claims

1. A method of hydrodefluorination of aromatic trifluoromethyl compounds of formula I 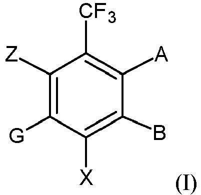
wherein A, B, X, G and Z are independently selected from the group comprising H, I, Br, F, Cl, CF₃, COOR, NO₂, N=N-R, SO₃R, CH₂R, OR, NR₂, CH₂NR₂, R-C=O, CN;
wherein R is H, C₁-C₈ alkyl, C₆-C₁₄ aryl, or C₂-C₁₀ heterocyclyl containing one to four heteroatoms independently selected from O, S, N;
in alkaline aqueous environment, said method comprising the steps of:
a) adding to an aqueous or colloid solution of the compound of formula I, at temperature within the range of 0 to 100 °C a mixture comprising:
- component A, which is an alloy containing aluminium (Al) acting as reducing agent and nickel (Ni) acting as hydrodefluoration catalyst, wherein the molar ratio of CF₃ : Al is within the range of 1 mol of CF₃ to 10 or more mol Al, and the molar ratio of CF₃ : Ni is within the range of 1 mol CF₃ to 4.6 or more mol Ni;
- component B, which is at least one alkali metal hydroxide or carbonate in molar ratio to Al 2:1 to 6:1, wherein the amount used corresponds to pH of the reaction mixture higher than 11;
b) stirring the resulting reaction mixture at a temperature within the range of 10 to 100 °C, for at least 60 minutes and at most 24 hours;
c) separating the nickel sludge from the aqueous phase of the reaction mixture;
d) adjusting pH of the separated aqueous phase of the reaction mixture to a value within the range of 6 to 9, which causes precipitation of insoluble aluminium salts which adsorb microparticles of nickel and organic compounds from the mixture, and separating the precipitate.

2. The method according to claim 1, wherein the component A is Raney Al-Ni alloy with weight ratio of Al:Ni = 1:1, i.e. 50 wt.% Al and 50 wt.% Ni, or nickel bronze, or alloys containing the metals CuAlNiFe and optionally Mn, preferably with molar ratio Al:Ni = 2:1 or higher, such as CuAl₁₀Ni₅Fe₄..

3. The method according to claim 1 or 2, wherein further a component C is added to the solution of compound of formula I, acting as an auxiliary reducing agent for reducing other reducible functional groups than CF₃ contained in the compound of formula I.

4. The method according to claim 3, wherein the component C is selected from the group comprising tetrahydroborates, secondary C3-C6 alcohols, elemental aluminium, iron, ferrous(II) sulfate, ferrous(II) chloride, magnesium, Devarda's Al-Cu-Zn alloy, Arnd's alloy Cu-Mg, hydrazine and salts thereof with formic, sulfuric or hydrochloric acids, phosphinic acid and salts thereof with alkali metals, and/or reducing saccharides.

5. The method according to any one of the preceding claims, wherein the component B is selected from the group comprising sodium hydroxide, potassium hydroxide, sodium carbonate, potassium carbonate, and mixtures thereof.

6. The method according to any one of the preceding claims, wherein the step (a) is preceded by a pre-treatment step comprising reduction of other reducible functional groups than CF₃ contained in the compound of formula I by applying as a reducing agent the component C together with an undissolved part of used component A.

7. The method according to any one of the preceding claims, wherein in step d) the pH value of the separated aqueous phase of the reaction mixture is adjusted to pH in the range of 6 to 9, using an acidic-reacting agent selected from the group comprising saturation by gas mixtures comprising CO₂, aqueous solution of sulfuric acid, aqueous solution of hydrochloric acid, aqueous solution of phosphoric acid, or aqueous solution of nitric acid.

## Patentansprüche

1. Verfahren zur Hydrodefluorierung von aromatischen Trifluormethylverbindungen der Formel I 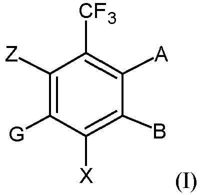
wobei A, B, X, G und Z unabhängig voneinander aus der Gruppe ausgewählt sind, die H, I, Br, F, Cl, CF₃, COOR, NO₂, N=N-R, SO₃R, CH₂R, OR, NR₂, CH₂NR₂, R-C=O, CN umfasst;
wobei R ist H, C1-C8-Alkyl, C6-C14-Aryl oder C2-C10-Heterocyclyl, das ein bis vier Heteroatome enthält, die unabhängig voneinander aus O, S, N ausgewählt sind;
in alkalischer wässriger Umgebung, wobei das Verfahren die folgenden Schritte umfasst:
a) Hinzufügen eine Mischung zu einer wässrigen oder kolloidalen Lösung der Verbindung der Formel I bei einer Temperatur im Bereich von 0 bis 100 °C, die Mischung umfassend:
- Komponente A, die eine Legierung ist, die Aluminium (Al) als Reduktionsmittel und Nickel (Ni) als Hydrodefluorierungskatalysator enthält, wobei das Molverhältnis von CF₃ : Al im Bereich von 1 Mol CF₃ zu 10 oder mehr Mol Al liegt und das Molverhältnis von CF₃ : Ni im Bereich von 1 Mol CF₃ zu 4,6 oder mehr Mol Ni liegt;
- Komponente B, die mindestens ein Alkalimetallhydroxid oder -carbonat im Molverhältnis zu Al von 2:1 bis 6:1 ist, wobei die verwendete Menge einem pH-Wert der Reaktionsmischung über 11 entspricht;
b) Rühren der resultierenden Reaktionsmischung bei einer Temperatur im Bereich von 10 bis 100 °C für mindestens 60 Minuten und höchstens 24 Stunden;
c) Abtrennen des Nickelschlamms von der wässrigen Phase des Reaktionsgemischs;
d) Einstellen des pH-Werts der abgetrennten wässrigen Phase des Reaktionsgemischs auf einen Wert im Bereich von 6 bis 9, wodurch unlösliche Aluminiumsalze ausgefällt werden, die Mikropartikel aus Nickel und organischen Verbindungen aus dem Gemisch adsorbieren, und Abtrennen des Niederschlags.

2. Verfahren nach Anspruch 1, wobei die Komponente A ist eine Raney Al-Ni-Legierung mit einem Gewichtsverhältnis von Al:Ni = 1:1, d. h. 50 Gew.-% Al und 50 Gew.-% Ni, oder Nickelbronze oder Legierungen, die die Metalle CuAlNiFe und optional Mn enthalten, vorzugsweise mit einem Molverhältnis Al:Ni = 2:1 oder höher, wie CuAl₁₀Ni₅Fe₄.

3. Verfahren nach Anspruch 1 oder 2, wobei der Lösung der Verbindung der Formel I auch eine Komponente C zugesetzt wird, die als Hilfsreduktionsmittel zur Reduzierung anderer reduzierbarer funktioneller Gruppen als CF₃ dient, die in der Verbindung der Formel I enthalten sind.

4. Verfahren nach Anspruch 3, wobei die Komponente C ausgewählt ist aus der Gruppe, die Tetrahydroborate, sekundäre C3-C6-Alkohole, elementares Aluminium, Eisen, Eisen(II)-sulfat, Eisen(II)-chlorid, Magnesium, Devardas Al-Cu-Zn-Legierung, Amds Legierung Cu-Mg, Hydrazin und dessen Salze mit Ameisen-, Schwefel- oder Salzsäure, Phosphinsäure und deren Salze mit Alkalimetallen und/oder reduzierende Saccharide umfasst.

5. Verfahren nach einem der vorhergehenden Ansprüche, wobei die Komponente B aus der Gruppe ausgewählt ist, die Natriumhydroxid, Kaliumhydroxid, Natriumcarbonat, Kaliumcarbonat und Mischungen davon umfasst.

6. Verfahren nach einem der vorhergehenden Ansprüche, wobei dem Schritt (a) ein Vorbehandlungsschritt vorangeht, der die Reduktion anderer reduzierbarer funktioneller Gruppen als CF₃ umfasst, die in der Verbindung der Formel I enthalten sind, indem als Reduktionsmittel die Komponente C zusammen mit einem ungelösten Teil der verwendeten Komponente A verwendet wird.

7. Verfahren nach einem der vorhergehenden Ansprüche, wobei in Schritt d) der pH-Wert der abgetrennten wässrigen Phase des Reaktionsgemischs auf einen pH-Wert im Bereich von 6 bis 9 eingestellt wird, wobei ein sauer reagierendes Mittel verwendet wird, das aus der Gruppe ausgewählt ist, die die Sättigung durch CO₂-umfassenden Gasgemische, wässrige Schwefelsäurelösung, wässrige Salzsäurelösung, wässrige Phosphorsäurelösung oder wässrige Salpetersäurelösung umfasst.

## Revendications

1. Procédé d'hydrodéfluoration de composés trifluorométhyle-aromatiques de formule I 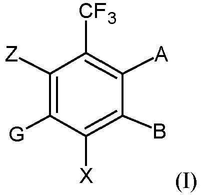
où A, B, X, G et Z sont indépendamment sélectionnés dans le groupe comprenant H, I, Br, F, Cl, CF₃, COOR, NO₂, N=N-R, SO₃R, CH₂R, OR, NR₂, CH₂NR₂, R-C=O , CN;
où R est H, alkyle en C1-C8, aryle en C6-C14 ou hétérocyclyle en C2-C10 contenant un à quatre hétéroatomes indépendamment sélectionnés parmi O, S, N;
en milieu aqueux alcalin, ledit procédé comprenant les étapes de:
a) ajouter à une solution aqueuse ou colloïdale du composé de formule I, à une température comprise entre 0 et 100°C, un mélange comprenant:
- le composant A, qui est un alliage contenant de l'aluminium (Al) agissant comme agent réducteur et du nickel (Ni) agissant comme catalyseur d'hydrodéfluoration, où le rapport molaire CF₃ : Al est compris dans la plage de 1 mole de CF₃ à 10 moles d'Al ou plus , et le rapport molaire CF₃ : Ni est compris dans la plage de 1 mole de CF₃ à 4,6 moles de Ni ou plus;
- le composant B, qui est au moins un hydroxyde ou carbonate de métal alcalin dans un rapport molaire à Al de 2:1 à 6:1, la quantité utilisée correspondant à un pH du mélange réactionnel supérieur à 11;
b) agitation du mélange réactionnel résultant à une température comprise entre 10 et 100 °C, pendant au moins 60 minutes et au plus 24 heures;
c) séparer les boues de nickel de la phase aqueuse du mélange réactionnel;
d) ajuster le pH de la phase aqueuse séparée du mélange réactionnel à une valeur comprise entre 6 et 9, ce qui provoque la précipitation de sels d'aluminium insolubles qui adsorbent les microparticules de nickel et de composés organiques du mélange, et séparer le précipité.

2. Procédé selon la revendication 1, où le composant A est un alliage Raney Al-Ni avec un rapport pondéral Al:Ni = 1:1, soit 50 % en poids d'Al et 50 % en poids de Ni, ou du bronze au nickel, ou des alliages contenant les métaux CuAlNiFe et éventuellement Mn, de préférence avec un rapport molaire Al:Ni = 2:1 ou supérieur, tel que CuAl₁₀Nl₅Fe₄.

3. Procédé selon la revendication 1 ou 2, où en outre un composant C est ajouté à la solution du composé de formule I, agissant comme agent réducteur auxiliaire pour réduire d'autres groupes fonctionnels réductibles que CF₃ contenus dans le composé de formule I.

4. Procédé selon la revendication 3, où le composant C est choisi dans le groupe comprenant les tétrahydroborates, les alcools secondaires en C3-C6, l'aluminium élémentaire, le fer, le sulfate ferreux (II), le chlorure ferreux (II), le magnésium, alliage de Devarda Al-Cu-Zn, alliage d'Arnd Cu-Mg, hydrazine et leurs sels avec les acides formique, sulfurique ou chlorhydrique, acide phosphinique et leurs sels avec des métaux alcalins, et/ou saccharides réducteurs.

5. Procédé selon l'une quelconque des revendications précédentes, où le composant B est choisi dans le groupe comprenant l'hydroxyde de sodium, l'hydroxyde de potassium, le carbonate de sodium, le carbonate de potassium et leurs mélanges.

6. Procédé selon l'une quelconque des revendications précédentes, où l'étape (a) est précédée d'une étape de prétraitement comprenant la réduction d'autres groupes fonctionnels réductibles que CF₃ contenus dans le composé de formule I en appliquant comme agent réducteur le composant C avec une partie non dissoute du composant A utilisé.

7. Procédé selon l'une quelconque des revendications précédentes, où à l'étape d) la valeur du pH de la phase aqueuse séparée du mélange réactionnel est ajustée à un pH compris dans la plage de 6 à 9, en utilisant un agent acidique choisi parmi le groupe comprenant la saturation par des mélanges gazeux comprenant du CO₂, une solution aqueuse d'acide sulfurique, une solution aqueuse d'acide chlorhydrique, une solution aqueuse d'acide phosphorique ou une solution aqueuse d'acide nitrique.
